# EUROPEAN PATENT APPLICATION

(11) **EP 3 798 320 A1**
(43) Date of publication of application: **31.03.2021**
(21) Application number: 20386048.1
(22) Date of filing: 25.09.2020
(51) Int. Cl.: C12Q 1/6844, G01N 33/543, G01N 33/569, B01L 3/00

(54) **DIAGNOSTIC CHIP FOR ANALYZING THE PRESENCE OF BACTERIA IN A SAMPLE**

(30) Priority: 25.09.2019 GR 20190100415
(71) Applicant: Nanoplasmas Private Company, 15341 Agia Paraskevi Attikis (GR)
(72) Inventor: GOGOLIDES, Evangelos, 15341 Agia Paraskevi Attikis (GR); TSEREPI, Angeliki, 15341 Agia Paraskevi Attikis (GR); TSOUGENI, Katerina, 15341 Agia Paraskevi Attikis (GR); ELLINAS, Kosmas, 15341 Agia Paraskevi Attikis (GR); KASTANIA, Athina, 15341 Agia Paraskevi Attikis (GR); KAPROU, Georgia, 15341 Agia Paraskevi Attikis (GR); LOUKAS, Christos-Moritz, 15341 Agia Paraskevi Attikis (GR)

(57) **Abstract**

A bacterium capture microfluidic device for ultrafast and highly sensitive analysis of the presence of bacteria in a liquid sample. The analysis is based on capture and lysis of viable bacteria on antibodies functionalized and lyophilized on chip, DNA amplification and visual detection.

## Description

### Field of the Invention

The present invention relates to the development of bacteria capture microfluidic devices for ultrafast and highly sensitive analysis of the presence of bacteria in a sample. These diagnostic lab-on-a-chip devices may be advantageous and applicable from medical to food and environmental sample analysis.

### State of the art and background of the invention

Diagnostics is a rapidly evolving field focusing on early stage prognosis, prediction and prevention of diseases, infections or other health risks. Despite the fact that laboratory tests represent 3% of the total healthcare spending, they influence more than 70% of the medical decisions worldwide. Most research and commercialization efforts have so far focused on the medical / health sector. However, food and water are also critical fields that require diagnostics, since more and more food-related diseases and serious poisoning outbreaks appear worldwide. Water is considered as 'food' under Regulation (EC) No 178/2002 of the European Parliament and of the Council laying down the general principles and requirements of food related laws. Food and water-borne infections are commonly caused by bacteria. Indeed, more than 90% of the cases of food poisoning are caused by 13 bacteria, including *Clostridium Perfringens*, *Staphylococcus Aureus*, *Vibrio* spp. (including V. cholerae and V. parahaemolyticus), *B. cereus*, *Salmonella* spp., *Clostridium Botulinum*, *Shigella* spp., toxigenic *E. coli* (ETEC and EHEC), and certain species of *Campylobacter*, *Yersinia*, *Listeria*, and *Aeromonas.* These bacteria are commonly found in many raw foods and if present even in small numbers they may cause illness. Furthermore, practically the same list of bacteria are also of interest in medical applications, where urine and blood samples are often analyzed to check the presence of such bacteria and prescribe the appropriate antibiotic treatment. The need for fast detection also applies in body fluids in order to prescribe bacteria specific antibiotics, rather than general purpose antibiotics.

As an example of the need for fast and accurate bacteria detection, we refer below the case of Legionella bacterium, which has recently been pinpointed by the World Health Organization (http://ecdc.europa.eu/en/datatools/Pages/home.aspx; http://www.who.int/mediacentre/factsheets/fs285/en/) as the highest health burden of all waterborne pathogens in the European Union and many outbreaks are reported around the globe every year. Legionella have been found in a wide range of systems including hot and cold water systems (both large systems serving public buildings and small systems in people's homes), cooling towers and some forms of air conditioning systems, on-board ships, airplanes and trains, water fountains, food misters, whirlpool spas and even in car washes (A. Edagawa, et al., Journal of Applied Microbiology, 2008, 105, 2104-2114). Infection occurs when breathing air containing water droplets contaminated with bacteria and is fatal in approximately 15-20% of the cases. Under the right conditions, Legionella bacteria can double in number every 90 minutes, meaning that a very low level of bacteria could become thousands of times higher in one or two days (N. Ishizaki, et al., Microbiology and Immunology, 2016, 60, 203-208). To prevent outbreaks and deaths, the European Centre for Disease Prevention and Control recommends to apply regular checks and appropriate control measures to man-made water systems to prevent cases of 'Legionnaires' disease at recreation centers, tourist accommodation sites, hospitals, long-term healthcare facilities or other settings, where sizeable populations may be exposed. Today, standard analysis methods (conventional bacterial culturing, such as ISO 11731 for Legionella) for most of bacteria (e.g. Salmonella, Campylobacter, and Legionella) need several days (∼ 3, 7, and 12, respectively) in specialized analytical laboratories to yield positive or negative results for potentially contaminated samples (M. Steinert, et al., Applied and Environmental Microbiology, 1997, 63, 2047-2053). This long delay between sampling and answer, and the resulting low frequency sampling, does not allow for early action to prevent pathogen outbreaks, in facilities of public interest.

Over the last decades, great effort has been made for more efficient water diagnostics for pathogens and faster analyses methods (J. Mairhofer, et al., Sensors, 2009, 9, 4804-4823). Nevertheless, two of the most challenging issues, including 1) limitations of sample preparation / preconcentration and 2) sensitivity of detection methods still remained unsolved. Therefore, simple, rapid, accurate, inexpensive, real-time, portable, and easy-to-use methods are needed for on demand detection of pathogens in water samples. This will require further advance and adaptation of new revolutionary technologies to facile rapid diagnosis of waterborne pathogens, and this is the opportunity and gap that the present invention achieved to fill in.

The advent of micro/nanotechnology, and especially microfluidics and laboratories on chip (lab-on-chip) brings many advantages such as reduced experimental time, smaller sample and reagent volumes, low contamination risk, automation, low cost and compatibility with downstream processes. (K. Kant, et al., Biotechnology Advances, 2018, 36, 4, 1003-1024). Lab-on-a-chip (LoC) technology enables the integrated processing of samples on a chip from sample preparation to final product (in case of product synthesis or purification) or final detection (in case of analysis). In recent years, intensive researches on microfluidic systems have generated powerful tools for diagnostic applications. These progresses are achieved due to advantages associated with miniaturization, i.e. improved sensitivity and specificity, automation, portability, versatility in design, multiple and parallel sample detection, minimal handling of hazardous materials, and time and cost savings (S. Kumar, et al., Biotechnology Journal, 2013, 8, 1267-1279; K.F. Lei, Journal of Laboratory Automation 2012, 17, 330-47; S. Tasoglu, et al., Chemical Society Reviews, 2013, 42, 5788-808). Additionally, all analytical processes, such as sample pre-treatment, separation, chemical reactions, and real-time quantification can be integrated into a single microfluidic platform for at site testing applications (R. Wang, et al., Analytica Chimica Acta, 2015, 853, 710-717).

Several microfluidic platforms have been reported for this purpose using various techniques (C. Duarte, et al., Biomedical Microdevices, 2013, 15, 821-830; K.F. Lei, Journal of Laboratory Automation, 2012, 17, 330-47; J. Mairhofer, et al., Sensors (Switzerland), 2009, 9, 4804-4823; S. Tasoglu, et al., Chemical Society Reviews, 2013, 42, 5788-808; M. Kim, et al., Biosensors and Bioelectronics, 2015, 74, 1011-1015; M. Jimenez, et al., Journal of Microbiological Methods, 2016, 126, 8-11; N. Yamaguchi, et al., Scientific Reports, 2017, 7, 3092; G. Huang, et al., Scientific Reports, 2017, 7: 6441). However, microfluidic-based methods for the detection of water or other fluid-born bacterial pathogens (Helen Bridle, Brian Miller, Marc P.Y. Desmulliez, Application of microfluidics in waterborne pathogen monitoring: A review, Water Research, 55, 2014, 256-271) are still challenging due to the limitations related to the integration of different crucial steps, such as sample pre-treatment, assay operations and detection on a single microfluidic chip (T.-H. Kim, et al., Analytical Chemistry, 2014, 86, 3841-3848). In addition, the requirement of small volume of sample in microfluidic channels might render certain hurdles in terms of desirable sensitivity, selectivity, and stability of the microfluidics sensors.

Among different isothermal DNA amplification techniques, LAMP has attracted significant attention due to its fast amplification, simple operation, robustness, and high sensitivity and specificity (M. Parida, et al., Reviews in medical virology, 2008, 18, 407-421). The specificity of LAMP is higher than PCR, since LAMP uses four to six different primers that bind to specific sites on the template strand (P. Craw, et al., Lab Chip, 2012, 12, 2469-2486). LAMP amplifies nucleic acid at temperatures between 60-66°C by leveraging Bst polymerase enzyme with high strand displacement activity (T. Notomi, et al., Nucleic Acids Research, 2000, 28, E63; N. A. Tanner, et al., Current protocols in molecular biology, 2014, 105, Unit 15.14).

With the significant development of various LoC devices (P. J. Asiello, et al., Lab Chip, 2011, 11, 1420-1430; L. Zanoli, et al., Biosensors, 2013, 3, 18; D.M. Tourlousse, et al., Biomedical Microdevices, 2012, 14, 769-778; X. Fang, et al., Analytical Chemistry, 2010, 82, 3002-3006; C. H. Wang, et al., Lab Chip, 2011, 11, 1521-1531), several diagnostic nucleic acid platforms have been developed and commercialized (Eicken Chemicals, SAS Loopamp Realtime, Turbidimeter LA-320C, http://www.uib.no/med/avd/ii/nyhetsbrev/2010/15/Turbidimeter.pdf); Eiken Chemicals, LA-500, http://loopamp.eiken.co.jp/e/products/la500/img/02.pdf); Meridianbioscience, Meridian illumipro-10™, http://www.meridianbioscience.com/Content/Assets/Files/2.1%20%20C.%20difficile %20Products/illumigene%20C.%20difficile/illumigene%20technology%20page/illum igene product folder.pdf); SMART, Smart-DART™ 8-Well V.3.0 http://diagenetix.com/smart-dart-platform/); Optigene, Genie II., http://www.optigene.co.uk/instruments/instrument-genie-ii/); Optigene, Genie III., http://www.optigene.co.uk/instruments/instrument-genie-iii/); QIAGEN, ESEQuant Tube Scanner, http://www.qiagen.com/resources/download.asp?id=3bf98195-8fe6-4b11-aadfb2499a920461&lang=en). The detection mechanism is usually fluorescence or turbidity, the cost per test varies from 2 - 80 $ and the reader cost varies from 2,5K to 25K $. Significant routine features are the detection time, the limit of detection (LOD), detection range, and the specificity. The LOD ranges from 10² -10⁵ CFU/mL and the detection time from 20 min to 6 h (Y. Sun, et al., Lab Chip, 2015, 15, 1898-1904; W. B. Valderrama, et al., Critical reviews in food science and nutrition, 2016, 56, 1519-1531; Y. Wang, et al., Comprehensive Reviews in Food Science and Food Safety, 2016, 15, 183-205; J. Y. Yoon, et al., Sensors (Basel), 2012, 12, 10713-10741). Most of the solutions that are currently available are mainly based on bacteria culture and are offered as services providing the sampling kit and the results are available after several days. The few existing kits (Industrial Test Systems, Inc., AquaVial Water Test Kit For Bacteria-Single Pack aqua safe) have very poor sensitivity that do not cover for example the legionella concentration limit that will be imposed by the new legislation *((<100cfu*/*100ml), EU directive: Brussels, 1.2.2018, Proposal for a DIRECTIVE OF THE EUROPEAN PARLIAMENT AND OF THE COUNCIL on the quality of water intended for human consumption (recast)).* The Lovibond® Hydrosense Legionella Field Test Kit with an immunoassay-based device is available from Novatech International (http://www.novatech-usa.com/Products/).

Our group recently presented two portable micro-nano-bio-system prototypes with two distinct chip designs for ultra-fast analysis of pathogens in food, to address the urgent problem of food-related disease outbreaks (G. Papadakis, et al., Biosensors and Bioelectronics, 2018, 111, 52-58). The first LoC used immunocapturing on magnetic beads and performs most of the handling steps manually off-chip, while the second LoC performed all sample handling on chip including a) bacteria capture, b) lysis, c) DNA amplification and d) acoustic detection of four different bacteria. In a recent work, we also demonstrated a modular, integrated LoC platform accommodating bacteria capture, thermal lysis, DNA purification, isothermal DNA amplification (HDA), and off-chip detection after collection of successive eluted DNA effluents using gel electrophoresis for performing sample preparation in less than 86 min (K. Tsougeni, et al., Sensors and Actuators B: Chemical, 2019, 288, 171-179).

In all microfluidic platforms an on-chip sample preparation microfluidic is typically connected to a sensing area or a sensor on a different area housed on the same substrate or heterogeneously integrated on a different substrate. There are several detection schemes, and sensors, such as electrochemical, capacitive, conductivity, and several optical sensors, which include fluorescence, turbidity, colorimetric based on fluorescence or optical density (absorbance) change. Colorimetric methods are easy to use and often can be observed by naked eye as a color change. Typically, such methods are based on fluorescence enhancement by nanoparticles in order to be easily observable by naked eye (L. Zheng, et al., Biosensors and Bioelectronics, 2019, 124-125, 143-149). Rarely, simple color change may also be used based on optical density; however in that case a significant optical path is needed (millimeters), which is not easy to use directly on a microfluidic chip which is a few tens to a few hundreds of microns (µm) deep. Hence, typically, samples are directed to some type of well or large microreactor, where the depth is enough to allow optical observation (K. Kadimisetty, et al., Biosensors and Bioelectronics, 2018, 109, 156-163; A. Sayad, et al., Biosensors and Bioelectronics, 2018, 100, 96-104). Therefore, sample preparation microfluidics are not used for detection via absorbance and color change.

### Summary of the invention

The present invention provides a diagnostic microfluidic chip for water and other liquid or solid surfaces analysis, comprising ultra-fast, sensitive, highly specific, and precise bacteria detection. Competitive advantages are demonstrated, such as low reagent consumption, low cost, portability and disposability, ability to discriminate live and dead pathogens, color change, storage at RT, and compliance with current legislation. The diagnostic lab on a chip is capable to detect, semi - quantify, discriminate various bacteria, including various Legionella spp. serotypes, and is applicable for detecting waterborne, fluid born and solid surface born bacteria.

### Brief description of the drawings

Figure 1 sketches the process flow for the fabrication of the diagnostic microfluidic chip for water and liquid or solid surface analysis.
Figure 2 (a, b) shows the simple two-step detection protocol for water-borne pathogens, such as Legionella. (a) a short (5 min) off-chip filtration step for sample concentration, starting from 200 ml artificially contaminated water and, 2) on-chip bacteria capture, lysis, DNA amplification and color change detection. The diagnostic chip comprises 2 microchannels involving negative (i.e. no cells or DNA template present in LAMP reactions) and positive samples.
Figure 3 shows (a) Agarose gel image of the colorimetric LAMP amplicons for various *L. Pneumophila* bacteria concentrations. (b) The absorbance ratio A430/A560 for various cell concentrations after on-chip colorimetric LAMP amplification.
Figure 4 shows the absorbance ratio A430/A560 of the samples in various depths from 100 µm to 500 µm, which is proportionate to the drop in pH occurring by the production of protons as the LAMP amplification progresses.
Figure 5 shows (a) data showing the capturing efficiency of five different bacteria *(E-coli*, *Salmonella*, *Listeria*, *B. Cereus*, *L. Pneumophilla)* on the chip, with the sample directly injected after sample preconcentration. (b) Gel electrophoresis image of DNA amplicons for 1000 cells after 30 min of LAMP performed at 65°C on chip. Identical performance of lysis and amplification is observed for on chip and bench top equipment.
Figure 6 (a) capturing efficiency of *Salmonella* on six different filters, (b) capturing efficiency of *L. Pneumophilla.*

### Detailed description of the invention

The present invention provides an advanced diagnostic microfluidic chip for ultra-fast, sensitive, specific, and precise analysis of the presence of bacteria in a sample. The analysis results are available within 1-2 hours, in contrast to standard laboratory methods that require several days.

Thus, the present invention provides a microfluidic chip for the analysis of the presence of a bacterium in a sample, wherein the chip comprises a microchannel on a polymeric surface, wherein the polymeric surface is nanotextured with an oxygen or fluorine or fluorocarbon containing plasma under anisotropic etching conditions in the presence of an etching inhibitor, wherein a bacterium-specific antibody is immobilized on the surface of the microchannel, and wherein the depth of the microchannel is from 100µm to 500µm.

The chip of the present invention can be used for the analysis of the presence of any bacterium. Preferably, the bacterium is selected from the list comprising Legionella spp, Clostridium perfringens, Staphylococcus aureus, Vibrio spp. (including V. cholerae and V. parahaemolyticus), B. cereus, Salmonella spp., Clostridium botulinum, Shigella spp., toxigenic E. coli (ETEC and EHEC), Campylobacter, Yersinia, Listeria, and Aeromonas. More preferably, the bacterium is selected from Legionella spp., and Salmonella spp.

The production of bacterium-specific antibodies as well as primers for the amplification of the DNA of a bacterium is well known to a person skilled in the art. The antibodies can be immobilized on the surface of the microchannel, for example, by adsorption combined with chemical reaction due to the reactive chemical groups created on the surface after the plasma treatment.

Preferably, the depth of the microchannel is from 300µm to 500µm.

The sample according to the present invention may originate from a liquid, such as water or a biological fluid, or from another source, such as a solid surface.

The analysis of the presence of a bacterium according to the presence invention is based on on-chip bacteria immunoaffinity capturing, followed by DNA amplification and detection by a simple color change. This dual specificity (immunoaffinity capturing on antibodies plus specific DNA amplification) offers high specificity, and in addition, it can even distinguish dead or living bacteria, which is extremely important when deciding the actions that should be taken. A unique feature of the present invention is that the colorimetric detection can be performed on the same microfluidic chip (where immunocapturing and DNA amplification take place), without the need to transport the sample to a deep well or other unit for detection. This is achieved by choosing appropriately the depth of the microchannel.

The microfluidic chip comprises typically an inlet and a waste well connected with a microfluidic channel. A hydrophobic valve connects the microfluidic channel to the waste well to prevent sample loss or waste backflow. The chip includes a control and a sample microfluidic channel. The chip is housed on a polymeric or polymer coated substrate. For example, the substrate can be glass coated with a polymeric film. Preferably, the polymer of the chip surface of the present invention belongs to the group of acrylic copolymers, cyclo olefin (COP), polystyrene (PS), poly(ether ether ketone) (PEEK) and polydimethylsiloxane (PDMS). More preferably, the polymer is poly(methyl methacrylate) (PMMA).

The diagnostic chip, embeds a nanotextured surface using gaseous plasma technology (K. Tsougeni, et al., Langmuir, 2010, 26, 13883-13891; K. Tsougeni, et al., Lab Chip, 2010, 10, 462-469; K. Tsougeni, et al., Lab on a Chip, 2016, 16, 120-131; K. Tsougeni, et al., ACS Applied Materials & Interfaces, 2015, 7, 14670-14681; K. Tsougeni, et al., Analytical and Bioanalytical Chemistry, 2012,403,2757-2764).

The nanotextured surface has a high surface area on which a high density of capture biomolecules is used to isolate bacteria with an extremely high specificity and efficiency. Following bacteria capture in situ, specific DNA amplification and colorimetric detection allow for optical decision on the presence of living bacteria with a high sensitivity (below 5 bacteria).

Typically, the diagnostic chips are nanotextured by treatment with gaseous plasma. Preferably, the surface is etched and nanotextured in oxygen or fluorine or fluorocarbon containing plasmas or their mixtures. Under conditions of varying ion energy, processing time, and temperature, various nanotexture geometries may be produced resulting in different increase of the surface area compared to a flat surface. The etching and nanotexturing of the surface may be carried out using a high density helicon or inductively coupled plasma reactor, or a reactive ion etcher, or other plasma etchers.

The etching has to be carried out in the presence of etching inhibitors. As disclosed in GR20050100473, WO2007031799, GR20080100404 and WO2009150479 the formation of nanotexture is caused by polymer etching with simultaneous deposition of unetchable inhibitors from the plasma reactor walls, or electrodes, or polymer etching which contains pre-existing such inhibitors in the polymer or on the organic polymer surface. In inductively coupled plasma reactors (ICP) and-or helicon wave reactors if the electrostatic fields are not shielded in the antenna, co-sputtering of dielectric material during etching creates etching inhibitors resulting in nanotexture formation (E. Gogolides, et al., Journal of Physics D: Applied Physics,2011,44,174021). Etching inhibitors are also created from the electrode or electrode clamping ring. In reactive ion etcher (RIE) machines sputtering of electrode material creates etching inhibitors. Changing plasma parameters -for a given plasma reactor- changes the nanotexture geometrical characteristics.

Etching must be conducted using directional reactive ions in Reactive Ion Etchers or High Density Plasma Etchers at low pressure with conditions of pressure ranges from 5 - 100 mT, of plasma power ranges from 100 - 1000 W, and of bias voltage -20 - 200V.

In the present invention the diagnostic chips are prefunctionalized with biomolecules, preferably lyophilized biomolecules, that permits convenient long-term storage (up to 18 months), facilitates their transport at ambient temperature and simplifies the process for end-user.

The diagnostic chip is highly specific for the targeted bacteria due to the antibody immunocapturing and the DNA amplification. For example, in the case of *Legionella* spp., the chips detect, quantify, and discriminate various *Legionella* serotypes e.g. *L. Pneumophila* serogroups 1-13, *L. Longbeachae* and *L. Bozemanii.* A double specificity is embedded in the chip due to 1) biomolecule used to functionalize the chip towards specific bacteria, and 2) Primers used amplify the DNA of the captured bacteria.

The chip design and operation include several innovations. In our previous works (K. Tsougeni, et al., Lab on a Chip, 2016, 16, 120-131) in order to maximize the bacteria capture efficiency we have been trying to maximize the bacteria capture area by increasing the chip length, which has the drawback of increasing chip volume, and thus the cost of reagents for DNA amplification. To reduce the chip volume one has to move to shallow microfluidics of a few tens of microns. Shallow microfluidics however make impossible the colorimetric detection on chip via absorbance due to the very small optical path (change of color cannot be detected by eye or camera). It has now been surprisingly found that when the depth of the microchannel lies within the claimed range it is possible to establish the outcome of the analysis by colorimetric detection, for example, by naked eye or camera. Preferably, the length of the microchannel is such that the volume of the chip is from 10 microliters to 60 microliters, so that the consumption of reagents is not increased.

Another aspect of the present invention is a process for the analysis of the presence of a bacterium in a sample by using the microfluidic chip as disclosed above, wherein the process comprises preconcentration of the sample, injection of the sample in the microfluidic chip and visual detection, for example, by color change. If the sample originates from a solid surface, a swab is used to collect the sample and then liquid is extracted from the swab after immersion in buffer and agitation using a vortex. Therefore solid surfaces samples are converted to liquid samples. Before the sample can be used in the microfluidic chip of the present invention, sample preconcentration needs to be made in order to reduce the sample volume from several hundred ml to a few hundred microliters. According to a preferred embodiment, filtration or centrifugation-based sample concentration protocol is developed. 20-500 mL of contaminated water or other liquid is centrifuged or filtered, causing the bacteria to form a bottom solid layer (pellet). The liquid layer (supernatant) is removed first and the remaining pellet is resuspended in 25 - 50 µl buffered solution. Finally, the resuspended pellet solution is injected in the pre-functionalized chip to instigate/initiate antibody-based bacteria capture. For the filtration typically 0.2micrometer filters are used. Preferred filters are sulfonate containing, or nylon and cellulose acetate (depending on the bacterium) and preferred filter dead volume is 50-500microliters.

The present inventors have also surprisingly found that flowing the sample with continuous flow into the chip may significantly reduce the bacteria capture efficiency, even at very small flowrates, although the surface area provided by the nanotexturing may be enough to capture the bacteria. This effect is more intense when the sample volume is above 100 microliters. Surprisingly, this effect can be eliminated by injecting the sample in batch mode as follows: A sample volume equal to the chip volume is introduced and is allowed to react with the antibodies for a time ranging from a few minutes to half an hour. After the bacteria have been bound to the antibody the sample is pushed to the waste and a second batch of equal volume is allowed to enter and react with the antibodies. This solution of sample introduction in 1-4 sample batches maintains the high efficiency of the chip, while keeping the chip volume low and the chip depth large enough for visual detection.

According to the present invention, bacteria capturing occurs on antibody-functionalized nanotextured chip surfaces and not on magnetic beads. No magnets are needed.

Following bacteria capture, a lysis buffer and a DNA amplification cocktail is introduced. Among the various amplification methods, isothermal amplifications are preferable. A preferred isothermal amplification method is the LAMP amplification. Detection can be made visually by color change detected by eye, or by camera.

The lowest limit of the on-chip capturing, DNA amplification followed by color detection is below 5 CFU. The sensitivity of the chips can be adjusted depending on the legislation limit for particular samples, for example, drinking water or irrigation water etc.

The unsurpassed sensitivity of less than 5 CFU is for example one order of magnitude more sensitive than the Legionella spp. concentration limit defined from the existing legislation for drinkable water (100 CFU /100ml of water).
A positive test result indicates that the bacteria are present in the sample above the detection limit of legislation.

A negative test result indicates that the bacteria are not detected or the number of bacteria in the sample are below the detection limit of legislation.

The diagnostic chip has advantages over current technologies by achieving: a reduction of analysis time by 99%; simplification of fabrication by using advanced chip technology, including use of a visual detection; eliminating the need for laborious and time consuming culturing/plating protocols and highly-trained personnel; cost-effectiveness, using simple bio-processes, and high specificity to the particular bacteria the chip is designed for.

The advantages of the present invention include simplicity; rapidity and steadiness over time, ability to discriminate live and dead pathogens, possibility of parallelization, portability (hand-held device that can be used at the point of need, i.e. the field of interest or a laboratory) and detection of bacteria in water or other fluids as well as bacteria from solid surfaces.

The diagnostic chips can be used in a wide variety of sectors including: education, food and beverage, hotels and spas, shipping, pools and whirlpool spas, offshore oil and gas, conventional and nuclear energy, hospitals, nursing homes and many more, milk and other food processing or selling facilities etc.

The present methodology paves the way to the creation of a new generation of products for rapid bacteria analysis for food safety and is a strong candidate for the point-of-care.

### Examples

### Example 1: Design and fabrication of the diagnostic LoC using pre-functionalized and freeze dried chips

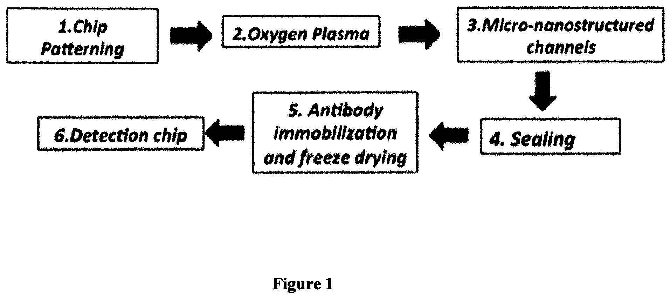

Figure 1 shows the process flow for the fabrication of the diagnostic microfluidic chip for water analysis. The chips are created on 4 mm thick acrylic polymer substrates. The method chosen for the prototyping was Computer Numerical Control (CNC) micromilling. The chip design and fabrication process are cost effective and apply the novel, fast (in the order of 15 min) plasma nanotexturing of polymer chip walls for highly efficient antibody binding capacity (K. Tsougeni, et al., Lab on a Chip, 2016, 16, 120-131; K. Tsougeni, et al., ACS Applied Materials & Interfaces, 2015, 7, 14670-14681; K. Tsougeni, et al., Langmuir, 2010, 26, 13883-13891). After the definition of the microchannel with micromilling, plasma nanotexturing through a stencil mask followed, so that surface roughening and functionalization by the plasma outside the microchannel was avoided and took place only inside the microchannel bottom wall. As a result, surfaces outside the microchannel were smooth, and good sealing of the microfluidic was easily achieved with a simple pressure sensitive adhesive lamination film. Antibodies are immobilized prior to use, and the prefunctionalized chip is subjected to freeze drying in order to greatly simplify the end-user task. The final diagnostic chip comprises 2 microchannels involving negative (i.e. no cells or DNA template present in LAMP reactions) and positive samples in order to record the negative and positive signal statistics and strengthen data interpretation. Microchannel depth is ∼500 µm, while roughness height is ∼ 5 µm.

### Example 2: The diagnostic LoC operation and validation protocol for water sample analysis

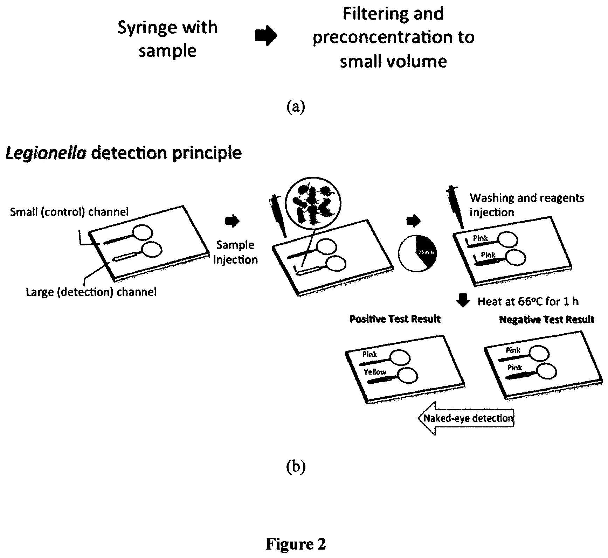

An outline of the two-step procedure is illustrated in Fig. 2: a) Filtration or centrifugation and resuspension to a small volume for pre-concentration, b) on-chip capture using freeze dried antibodies, on-chip lysis of captured cells, on-chip LAMP for DNA amplification, followed by color change detection. The sequence followed in the chip is: i) water sample injection (total volume 25 - 100 µL), ii) PBS buffer injection to wash the unbound bacteria (total volume 30 µL), iii) LAMP reagent injection (total volume 24 µL), iv) chemical lysis with triton (included in the LAMP reaction) for 10 min, v) heating for 60 min @ 65°C, vi) naked-eye color change detection. The outcome of the colorimetric LAMP is verified by the color change from pink (negative, no cells) to yellow (positive) observed through naked eye.

A *Legionella* diagnostic chip was used combining on-chip bacteria immunoaffinity capturing, chemical lysis and DNA isothermal amplification, all three in a single microfluidic chamber, followed by a simple color change detection of the presence of Legionella bacterial DNA in a sample. A sample is taken, prepared according to the specific test procedure (see example 1 above), and the sample is dispensed onto the inlet. The presence of bacterial DNA causes the "Positive Line" to turn yellow in color. A "Negative Control Line" is included which always remains pink on successful completion of the test.

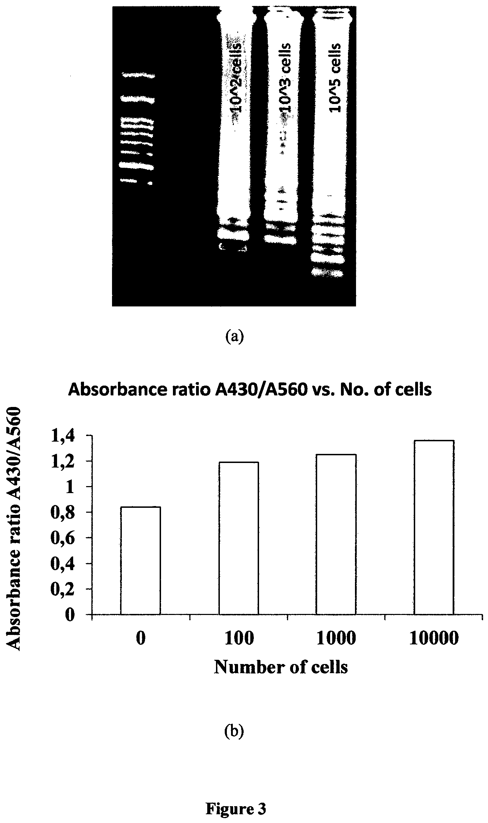

To demonstrate that the LoC can operate with different concentration samples, we include data showing the performance using different concentration after pre-concentration of water samples below and above the legionella concentration limit imposed by the new EU legislation. The data for the positive results show a shift of yellow color from faint to bright yellow by increasing the number of cells up to 10⁴ CFUs. After 10⁴ CFUs there is no significant difference indicating that saturation is reached. The results were further validated by gel electrophoresis. The large color difference between negative and positive samples promises a very sensitive naked eye detection even down to a few cells. We note that the lowest detection limit is 5 CFUs. Furthermore, in order to semi-quantify the obtained results, the absorbance of the samples is measured and the absorbance ratio A430/A560 is calculated (Figure 3) which is proportionate to the drop in pH occurring by the production of protons as the LAMP amplification progresses.

### Example 3: Depth of Legionella detection chips

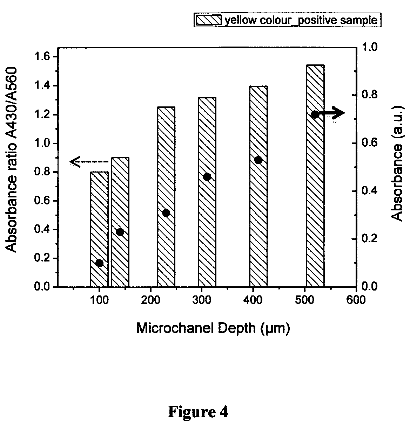

The same procedure as in example 2 above (absorbance measurements across the microchannels) was followed for microchannels of increasing depth (from 100 to 500 µm). This is necessary in order to optimize the color change by semi-quantification of the results as a function of the microchannel depth. The absorbance of the samples was measured in various depths from 100 µm to 500 µm and the absorbance ratio A430/A560 was calculated (Figure 4), which is proportionate to the drop in pH occurring by the production of protons as the LAMP amplification progresses. It is proven that the absorbance ratio A430/A560 increases versus the increased microchannel depth. A microchannel depth of 500 µm was chosen that combines both a high absorbance ratio and a small reagent volume (for minimization of the cost).

### Example 4: Detection of Salmonella, e-coli, Listeria, B. Cereus

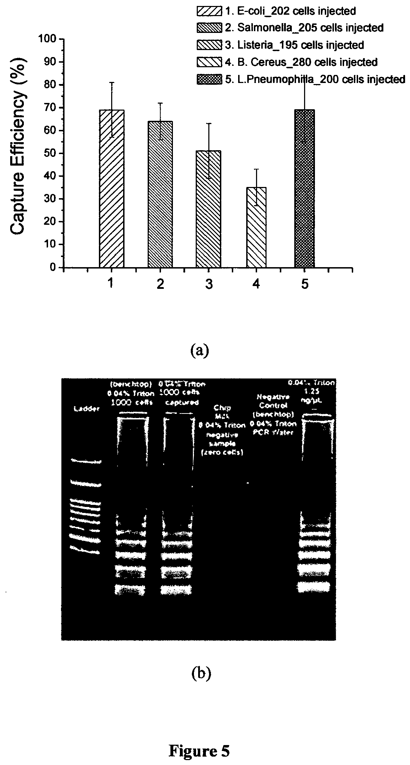

The same chip described in Examples 2 & 3, using an appropriate, however different from above recognition antibody, and different primers for DNA amplification, was applied for other bacteria from the list of the common pathogens including *Salmonella, E-coli, Listeria* and *B. Cereus.* We have used the chip to capture, lyse chemically, and amplify DNA for these bacteria, demonstrating the generic nature of our approach for different bacteria. In Fig. 5 (a), we include data showing the capturing efficiency of five different bacteria (*E-coli, Salmonella, Listeria, B. Cereus, L. Pneumophilla)* on the chip, with the sample directly injected after sample preconcentration. The capture efficiency depends on the bacterium and is between ∼ 35% and 70% (highest efficiency was observed for *E-coli*, *Salmonella*, *and L. Pneumophilla).* Chemical lysis was done using 0.04% (v/v) Triton X-100 at room temperature for 10 min, followed by DNA amplification. In Fig. 5 (b) we demonstrate on-chip DNA amplification for *Salmonella.*

### Example 5: Choice of Filters for different bacteria (L. Pneumophilla, Salmonella) for sample pre-concentration

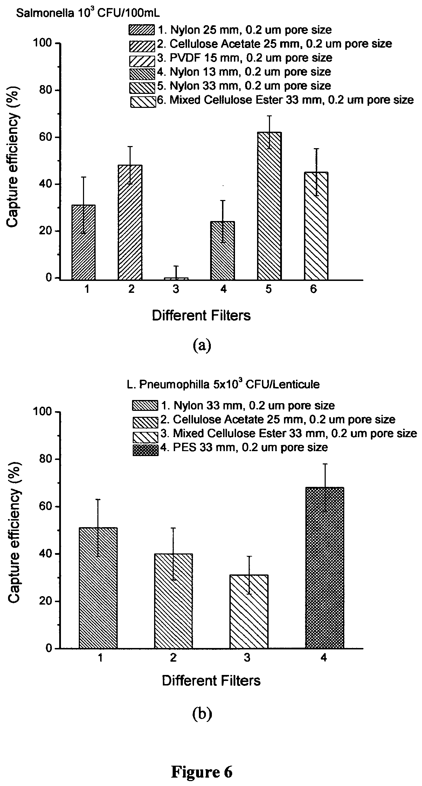

Filtration-based sample pre-concentration protocol, as described in Example 2, is demonstrated for two bacteria, *L. Pneumophilla* and *Salmonella.* 20 mL of contaminated water or other liquid is filtered on a variety of 0.2micrometer filters, causing the bacteria to form a bottom solid layer (pellet). Fig. 6 (a) shows the capturing efficiency of *Salmonella* on six different filters, while Fig. 6 (b) shows the capturing efficiency of *L. Pneumophilla.* It is observed that preferred filters are sulfonate containing or nylon for *L. Pneumophilla,* and nylon, cellulose acetate or cellulose ester for *Salmonella.* Preferred filter dead volume is 50-500microliters.

### Example 6: Detection of Legionella spp. after swabbing of surface samples

Recovering bacteria from other samples except liquids, such as solid surfaces was also tested, demonstrating the generic nature of our approach. A cotton swab bud was used to collect the bacteria from the surface. The sampling area was ∼10 cm². The bacteria were recovered by dipping the swap into an extracting solution and vortexing. Then, the same procedure as described in examples 2, 3 was followed. We used this procedure to sample bacteria from surfaces, e.g. air condition filters and we analyzed 8 samples. The samples were analyzed, and a visual color inspection showed that 3 samples were positive and 5 were negative in accordance with the ISO 11731 (2017) method.

## Claims

1. A microfluidic chip for the analysis of the presence of a bacterium in a sample, wherein the chip comprises a microchannel on a polymeric surface, wherein the polymeric surface is nanotextured with an oxygen or fluorine or fluorocarbon containing plasma under anisotropic etching conditions in the presence of an etching inhibitor, wherein the chip comprises a bacterium-specific antibody on the surface of the microchannel and wherein the depth of the microchannel is from 100µm to 500µm.

2. Use of a microfluidic chip according to claim 1 for the analysis of the presence of a bacterium in a sample.

3. Use according to claim 1 or 2, wherein the bacterium is selected from *Legionella* spp., *Clostridium Perfringens*, *Staphylococcus Aureus*, *Vibrio*, *B. cereus*, *Salmonella* spp., *Clostridium Botulinum*, *Shigella,* toxigenic *E. coli*, *Campylobacter*, *Yersinia*, *Listeria*, and *Aeromonas.*

4. Use according to claim 3, wherein the analysis is carried out by DNA amplification, and preferably by loop-mediated isothermal DNA amplification.

5. Use according to any one of claims 2 to 4, wherein the presence of the bacterium is assessed on the microfluidic chip by visual detection through color change.

6. A process for the analysis of the presence of a bacterium in a sample, wherein the process comprises the steps of
a) preconcentrating the sample,
b) injecting the sample in the microfluidic chip of claim 1,
c) performing DNA amplification on the microfluidic chip
c) assessing on the microfluidic chip the presence of the bacterium by visual detection through color change.

7. The process according to claim 6, wherein the preconcentration of the sample is carried out by centrifugation or by filtration.

8. The process according to claim 7, wherein the filter used in the filtration is a nylon filter, a cellulose acetate filter or a sulfonate-containing filter.

9. The process according to any one of claims 6 to 8, wherein the injection of the sample is carried out in batches, wherein the volume of each batch equals the volume of the microchannel of the microfluidic chip.

10. The process according to any one of claims 6 to 9, wherein the bacterium is selected from *Legionella* spp., *Clostridium Perfringens*, *Staphylococcus Aureus*, *Vibrio*, *B. cereus*, *Salmonella* spp., *Clostridium Botulinum*, *Shigella*, toxigenic *E. coli*, *Campylobacter*, *Yersinia*, *Listeria*, and *Aeromonas.*
